# EUROPEAN PATENT APPLICATION

(11) **EP 2 368 505 A1**
(43) Date of publication of application: **28.09.2011**
(21) Application number: 11250370.1
(22) Date of filing: 24.03.2011
(51) Int. Cl.: A61B 17/34

(54) **Portal apparatus with a tubular seal device**

(30) Priority: 25.03.2010 US 317317 P; 04.02.2011 US 21057
(71) Applicant: Tyco Healthcare Group LP, New Haven, CT 06511 (US)
(72) Inventor: Radziunas, Jeffrey P., Wallingford, CT 06492 (US)
(74) Representative: Soames, Candida Jane

(57) **Abstract**

A surgical portal apparatus includes a portal housing, a portal member, and an elongated seal mounted within the portal housing. The portal housing defines a housing opening for reception of a surgical object and the portal member extends from the portal housing. The elongated seal includes an outer wall segment and an inner seal segment. The outer wall segment has first and second ends that are mechanically coupled to the portal housing. The inner seal segment is disposed within the outer wall segment and depends radially inwardly relative to the longitudinal axis. The inner seal segment has inner surfaces that define a seal passage and is adapted to establish a substantial sealed relation with the surgical object. The outer wall segment includes a flexible material to permit deflection of the outer wall segment and corresponding movement of the inner seal segment in at least a radial direction with respect to the longitudinal axis.

## Description

### CROSS REFERENCE TO RELATED APPLICATION

The present application claims the benefit of and priority to U.S. Provisional Application Serial No. 61/317,317 filed on March 25, 2010, the entire contents of which are incorporated herein by reference.

### BACKGROUND

### Technical Field

The present disclosure relates to a portal apparatus adapted to allow the introduction of surgical instrumentation into a patient's body. In particular, the present disclosure is directed to a portal apparatus including a seal assembly adapted to establish a substantial sealed relation with a surgical object.

### Description of the Related Art

In laparoscopic procedures, surgery is performed in the interior of the abdomen through a small incision; in endoscopic procedures, surgery is performed in any hollow viscous of the body through narrow tubes or cannula inserted through a small entrance incision in the skin. Laparoscopic and endoscopic procedures generally require that any instrumentation inserted into the body be sealed, i.e. provisions must be made to ensure that gases do not enter or exit the body through the incision as, for example, in surgical procedures in which the surgical region is insufflated. Moreover, laparoscopic and endoscopic procedures often require the surgeon to treat organs, tissues, and vessels far removed from the incision, thereby requiring that any instruments used in such procedures be relatively long and narrow. Generally in the context of insufflatory surgical procedures, a substantially fluid-tight seal about an instrument being introduced within the portal is desirable.

### SUMMARY

Accordingly, the present disclosure is directed to a surgical portal apparatus including a portal housing, a portal member, and an elongated seal that is mounted within the portal housing. The portal housing defines a housing opening for reception of a surgical object and the portal member extends from the portal housing. The portal member defines a longitudinal axis and has a longitudinal passageway that is in general alignment with the housing opening for passage of the surgical object. In addition, the portal member is dimensioned to pass through tissue to provide access via the longitudinal passageway to an underlying operative site. The elongated seal includes an outer wall segment and an inner seal segment. The outer wall segment has first and second ends that are mechanically coupled to the portal housing. The inner seal segment is disposed within the outer wall segment and depends radially inwardly relative to the longitudinal axis. The inner seal segment has inner surfaces that define a seal passage and is adapted to establish a substantial sealed relation with the surgical object. The outer wall segment includes a flexible material to permit deflection of the outer wall segment and corresponding movement of the inner seal segment in at least a radial direction with respect to the longitudinal axis during offset manipulation of the surgical object within the seal passage.

In embodiments, the portal housing may include an outer wall and an inner wall within the outer wall. The inner wall may define a seal chamber, such that, the seal may be at least partially enclosed within the seal chamber. The inner wall may be dimensioned to engage the outer wall segment of the seal upon deflection of the outer wall segment a predefined radial distance.

In embodiments, the outer wall and the seal passage of the inner seal segment may be coaxially arranged with respect to the longitudinal axis. The inner surfaces of the inner seal segment define a central aperture. The outer wall segment is dimensioned to permit deflection of the outer wall segment and corresponding movement of the inner seal segment in at least an axial direction with respect to the longitudinal axis during manipulation of the surgical object within the seal passage.

In embodiments, the outer wall segment of the seal may be substantially tubular. The inner seal segment may be substantially planar and arranged in a general orthogonal relation with respect to the longitudinal axis. It is contemplated that the outer segment and the inner seal segment may be monolithically formed. It is also contemplated that a fluid may be disposed within the seal chamber external of the seal, such that, the fluid is dimensioned to restrict radial movement of the outer wall segment of the seal.

### BRIEF DESCRIPTION OF THE DRAWINGS

Various embodiments of the present disclosure are described herein with reference to the drawings wherein:

**FIG. 1** is a perspective view of a surgical portal apparatus including an elongated seal (in phantom) in accordance with the present disclosure;

**FIG. 2** is a side cross-sectional view of the elongated seal of **FIG. 1****;**

**FIG. 3** is a side cross-sectional view similar to the view of **FIG. 2** illustrating introduction of a surgical object through the elongated seal; and

**FIG. 4** is an enlarged side cross-sectional view of a portion of **FIG. 3** illustrating movement of the elongated seal and the surgical object.

### DETAILED DESCRIPTION

The portal apparatus of the present disclosure contemplates the introduction of various types of instrumentation adapted for insertion through a trocar and/or cannula assembly and incorporates a seal assembly adapted to maintain a substantially fluid-tight interface about the instrument to help preserve the atmospheric integrity of a surgical procedure from gas and/or fluid leakage. This feature of the present disclosure minimizes the entry and exit of gases and/or fluids to/from the body cavity.

Examples of instrumentation include, but are not limited to, clip appliers, graspers, dissectors, retractors, staplers, laser probes, photographic devices, endoscopes and laparoscopes, tubes, and the like. Such instruments will collectively be referred to as "instruments", "instrumentation" or "surgical objects" which also may include the hand of a clinician.

The portal apparatus may be any suitable cannula assembly used in laparoscopic or arthroscopic procedures. The portal apparatus may also be adapted to receive the hand of a surgeon during, e.g., a minimally invasive laparoscopic hand assisted procedure.

In the following description, as is traditional, the term "proximal" or "trailing" refers to the portion of the device closer to the operator while the term "distal" or "leading" refers to the portion of the device further from the operator.

Specifically, the portal apparatus incorporates a seal assembly which may deflect in one or both of a radial and longitudinal direction during insertion and/or manipulation of the instrumentation therein. This feature of the present disclosure minimizes the entry and exit of gases and/or fluids to/from the body cavity.

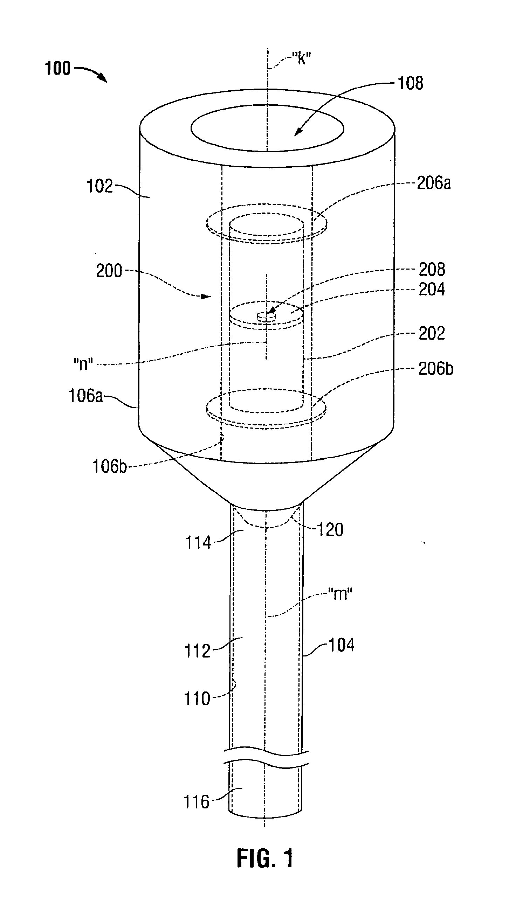

Referring now to the drawings, in which like reference numerals identify identical or substantially similar parts throughout the several views, **FIG. 1** illustrates an exemplary embodiment of the portal apparatus **100** in accordance with the principles of the present disclosure. Portal apparatus **100** may be a laparoscopic cannula assembly utilized in conjunction with a laparoscopic surgical procedure where the peritoneal cavity is insufflated with a suitable gas, e.g., CO₂, to raise the cavity wall from the internal organs therein. The cannula assembly may be used with an obturator assembly (not shown) which is a sharp pointed instrument positionable within the passageway of the cannula assembly. The obturator assembly is utilized to penetrate the abdominal wall and then subsequently removed from the cannula assembly to permit introduction of the surgical instrumentation utilized to perform the procedure. In the alternative, portal apparatus **100** may be an arthroscopic cannula assembly used in connection with an arthroscopic surgical procedure.

Portal apparatus **100** includes portal housing **102** and elongated portal member 104 extending from the portal housing **102.** Portal housing **102** may include multiple housing segments connected to each other via convention means or may be a single component integrally or monolithically formed. Portal housing **102** has inner housing wall **106** defining housing passage **108** coaxially arranged about a longitudinal housing axis **"k"** extending through the portal housing **102.** Inner housing wall **106** is dimensioned to receive a surgical object or instrument (not shown) and laterally confine the instrument within portal housing **102.** Inner housing wall **106** may be generally circular in cross-section or may assume other cross-sectional shapes.

Portal member **104** may be a sleeve member defining a longitudinal portal axis **"m"** extending along the length of the portal member **104.** Longitudinal portal axis **"m"** of portal member **104** may be in general longitudinal alignment with longitudinal housing axis **"k".** Portal member **104** includes outer sleeve wall **110** defining an internal longitudinal opening **112** extending from proximal or trailing end **114** through distal or leading end **116** of the portal member **104.** Longitudinal opening **112** of portal member **104** is in general longitudinal alignment with central housing passage **108** of portal housing **102** to define a common longitudinal passageway **108, 112** through portal apparatus **100** for passage of the surgical object. Portal member **102** may be a separate component connected to portal housing **102** or may be monolithically formed with the portal housing **102.** Portal member **104** and portal housing **102** may be releasably connected through a variety of mechanisms including, e.g., through a bayonet lock, threaded connection, or the like.

Portal member **104** may be formed of stainless steel or other rigid materials such as a polymeric material or the like. Portal member **104** may be clear or opaque. The diameter of portal member **104** may vary, but typically ranges from about **3** to about **15** mm when used in a laparoscopic or arthroscopic technique. If used in a hand assisted minimally invasive approach, the diameter of portal member may be substantially greater than **15** mm.

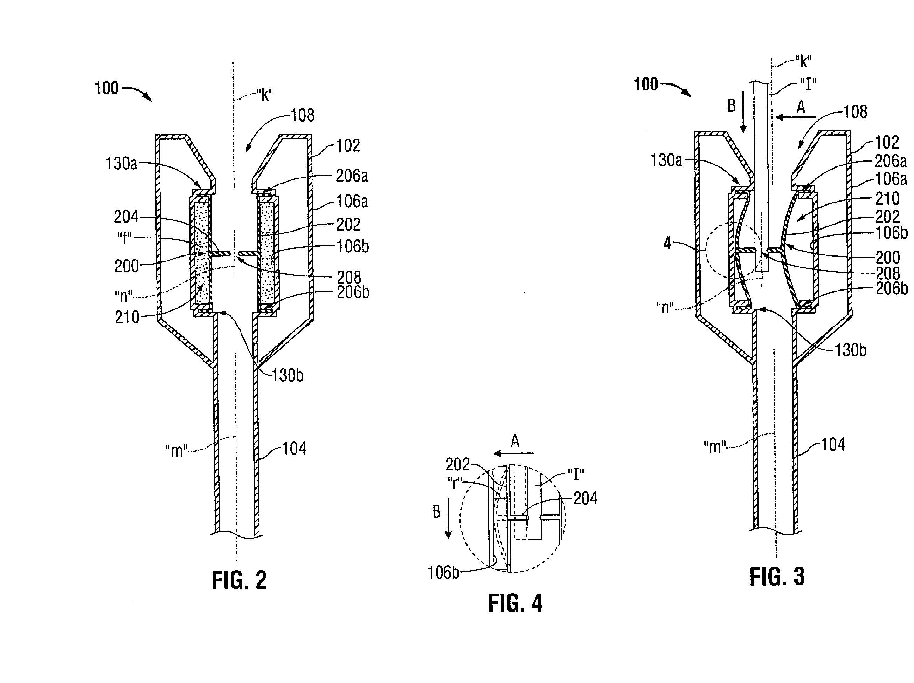

Referring now to **FIGS. 2-4****,** in conjunction with **FIG. 1****,** portal apparatus **100** further includes an elongated seal **200** defining a longitudinal axis **"n"** extending along the length of the seal **200.** The elongated seal **200** is mounted within the portal housing **102** and includes an outer wall segment **202** and an inner seal segment **204** that are coupled together in a monolithically formed manner. It should be noted that the inner seal segment **204** may be coupled to outer wall segment **202** by any suitable attaching means, however, it is essential that the attaching means provides a suitable seal to prevent any gases (from the patient's body) to pass therethrough.

In embodiments, portal housing **102** includes an outer wall **106a** and an inner wall 106b within the outer wall **106a.** The inner wall **106b** may define a seal chamber **210,** such that, the elongated seal **200** may be at least partially enclosed within the seal chamber **210.** The inner wall **106b** may be dimensioned to engage the outer wall segment **202** of the seal **200** upon deflection of the outer wall segment **202.** The inner wall **106b** and the outer wall segment **202** define a predefined radial distance **"r"** (as shown in **FIG. 4****)** when the seal is in an aligned position with the longitudinal axis **"k".**

It is envisioned that outer wall segment **202** of the seal **200** may have, for example, but not limited to, a substantially tubular shape. The inner seal segment **204** may be substantially planar and may be arranged in a general orthogonal and/or coaxial relation with respect to the longitudinal axis **"n".** However, as it will be described further below, the outer wall segment **202** and the inner seal segment **204** may be manipulated in different directions. It is also contemplated that a fluid medium **"F"** may be disposed within the seal chamber **210** external of the seal **200,** such that, the fluid medium **"F"** is dimensioned to restrict substantial radial movement of the outer wall segment **202** of the seal **200.** The fluid medium **"F"** may be, for example, but not limited to, a saline solution, air, or water.

Outer wall segment **202** has first and second ends **206a** and **206b,** respectively, which are mechanically coupled to the portal housing **102.** The first and second ends **206a** and **206b** protrude radially outwards from the outer wall segment **202** in a flange-like manner. The flange-like configuration allows the first and second ends **206a** and **206b** to be mechanically coupled and positioned within grooves **130a** and **130b,** respectively, of the portal housing **102.** Grooves **130a** and **130b** are essentially cavities formed along the length of inner wall **106b** and configured to facilitate securement of seal **200** within the portal housing **102.** First and second ends **206a** and **206b** may be affixed to grooves **130a** and **130b** by friction fitting, gluing, press fitting, snap fitting, or any other means.

When elongated seal **200** is in an unbiased position, the inner seal segment **204** is disposed within the outer wall segment **202** and depends radially inwardly along the longitudinal axis **"n"** of seal **200** and relative to the longitudinal axes **"k"** and **"m".** The inner seal segment **204** has inner surfaces that define a seal passage or a central aperture **208.** The seal passage **208** of the inner seal segment **204** is adapted to establish a substantial sealed relation with the surgical object **"I",** when surgical object **"I"** is introduced and positioned within the seal passage **208.** Thus, the gases from the patient's body are prevented from escaping the distal portion of portal member **104** and into the atmosphere. The inner seal segment **204** may be substantially planar and may extend in orthogonal relation to the longitudinal axis "k" when in an at rest condition.

The materials of fabrication of seal **200** may include a suitable elastomeric material whereby the seal passage **208** conforms to establish the seal about the surgical instrument **"I".** One suitable seal material which may be adapted for incorporated seal **200** is disclosed in commonly assigned U.S. Patent No. 6,482,181 to Racenet et al., the entire contents of which are hereby incorporated by reference herein. The seal disclosed in the Racenet '**181** patent includes an elastomeric material (such as isoprene or natural rubber) and at least one layer of fabric material. The fabric material may be any suitable fabric, for example, A SPANDEX material containing about **20**% LYCRA and about **80**% NYLON available from Milliken. The elastomeric material may be adhered to or embedded within the fabric material. Elongated seal **200** may be coated with a hydrophilic coating to facilitate passage of the surgical object **"I".**

Turning now to **FIGS. 3** and **4****,** the outer wall segment **202** includes a flexible material, for example, an elastomeric material, as mentioned above, to permit deflection of the outer wall segment **202** and corresponding movement of the inner seal segment **204.** The outer wall segment **202** and the corresponding movement of the inner seal segment **204** may deflect in a radial direction **"A"** and a radial distance **"r"** with respect to the longitudinal axis **"k"** during offset manipulation of the surgical object/instrument **"I"** within the seal passage **212.** The surgical object or instrument **"I"** may be manipulated in the radial direction **"A",** axial direction **"B",** and/or angular rotation of longitudinal axis **"n"** relative to the longitudinal axis **"k".** This freedom of flexibility and movement allows a clinician to manipulate the surgical object **"I",** within the radial distance **"r",** while at the same time, maintaining a seal relation with the seal **200** and surgical object **"I".** In embodiments, the outer wall **106a** and the seal passage **212** of the inner seal segment **204** may be coaxially arranged with respect to the longitudinal axis **"n".**

In one embodiment, portal housing **102** may also include a zero closure valve **120** disposed in mechanical cooperation within housing **102 (****FIG. 1****).** Zero closure valve **120** may be, e.g., a duckbill valve, slit valve, trumpet valve or the like adapted to provide a substantially fluid-tight seal in absence of a surgical object.

During use, instrument **"I"** is introduced in a leading direction depicted by arrow **"A".** As the instrument **"I"** is introduced in the leading direction **"A",** the inner seal segment **204** is influenced such that the instrument **"I"** can pass therethrough. That is, the inner seal segment **204** contours the instrument **"I"** to facilitate in providing a substantial seal, thus preventing insufflated air and/or gases to escape a peritoneal cavity of a patient.

Referring back to **FIG. 3****,** the instrument seal is shown having instrument **"I"** moved in a combination directions, for example, but not limited to, longitudinal direction **"A"** and axial direction **"B".** The instrument is inserted through seal device **120** and may ultimately be removed from seal device **120** by pulling the instrument proximally until the entire instrument is removed from portal apparatus **100** and elongated seal **200.**

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A surgical portal apparatus, which comprises:
   a portal housing defining a housing opening for reception of a surgical object;
   a portal member extending from the portal housing and defining a longitudinal axis and having a longitudinal passageway in general alignment with the housing opening for passage of the surgical object, the portal member dimensioned to pass through tissue to provide access via the longitudinal passageway to an underlying operative site; and
   an elongated seal mounted within the portal housing, the seal including:
      an outer wall segment having first and second ends mechanically coupled to the portal housing;
      an inner seal segment disposed within the outer wall segment and depending radially inwardly relative to the longitudinal axis, the inner seal segment having inner surfaces defining a seal passage adapted to establish a substantial sealed relation with the surgical object; and
      the outer wall segment comprising a flexible material to permit deflection of the outer wall segment and corresponding movement of the inner seal segment in at least a radial direction with respect to the longitudinal axis during offset manipulation of the surgical object within the seal passage.
**2.** The surgical portal apparatus according to paragraph **1** wherein the portal housing includes an outer wall and an inner wall within the outer wall, the inner wall defining a seal chamber, the seal being at least partially enclosed within the seal chamber.
**3.** The surgical portal apparatus according to paragraph **2** wherein the inner wall is dimensioned to engage the outer wall segment of the seal upon deflection of the outer wall segment a predefined radial distance.
**4.** The surgical apparatus according to paragraph **3** wherein the outer wall and the seal passage of the inner seal segment are coaxially arranged with respect to the longitudinal axis.
**5.** The surgical apparatus according to paragraph **4** wherein the inner surfaces of the inner seal segment define a central aperture, the central aperture being the seal passage.
**6.** The surgical apparatus according to paragraph **1** wherein the outer wall segment is dimensioned to permit deflection of the outer wall segment and corresponding movement of the inner seal segment in at least an axial direction with respect to the longitudinal axis during manipulation of the surgical object within the seal passage.
**7.** The surgical apparatus according to paragraph **6** wherein the outer wall segment of the seal is substantially tubular.
**8.** The surgical apparatus according to paragraph **7** wherein the inner seal segment is substantially planar and is arranged in general orthogonal relation with respect to the longitudinal axis.
**9.** The surgical apparatus according to paragraph **8** wherein the outer segment and the inner seal segment are monolithically formed.
**10.** The surgical apparatus according to paragraph **2** including a fluid disposed
   within the seal chamber external of the seal, the fluid dimensioned to restrict radial movement of the outer wall segment of the seal.

## Claims

1. A surgical portal apparatus, which comprises:
a portal housing defining a housing opening for reception of a surgical object;
a portal member extending from the portal housing and defining a longitudinal axis and having a longitudinal passageway in general alignment with the housing opening for passage of the surgical object, the portal member dimensioned to pass through tissue to provide access via the longitudinal passageway to an underlying operative site; and
an elongated seal mounted within the portal housing, the seal including:
an outer wall segment having first and second ends mechanically coupled to the portal housing;
an inner seal segment disposed within the outer wall segment and depending radially inwardly relative to the longitudinal axis, the inner seal segment having inner surfaces defining a seal passage adapted to establish a substantial sealed relation with the surgical object; and
the outer wall segment comprising a flexible material to permit deflection of the outer wall segment and corresponding movement of the inner seal segment in at least a radial direction with respect to the longitudinal axis during offset manipulation of the surgical object within the seal passage.

2. The surgical portal apparatus according to claim **1** wherein the portal housing includes an outer wall and an inner wall within the outer wall, the inner wall defining a seal chamber, the seal being at least partially enclosed within the seal chamber.

3. The surgical portal apparatus according to claim **2** wherein the inner wall is dimensioned to engage the outer wall segment of the seal upon deflection of the outer wall segment a predefined radial distance.

4. The surgical apparatus according to claim **3** wherein the outer wall and the seal passage of the inner seal segment are coaxially arranged with respect to the longitudinal axis.

5. The surgical apparatus according to claim **4** wherein the inner surfaces of the inner seal segment define a central aperture, the central aperture being the seal passage.

6. The surgical apparatus according to any preceding claim wherein the outer wall segment is dimensioned to permit deflection of the outer wall segment and corresponding movement of the inner seal segment in at least an axial direction with respect to the longitudinal axis during manipulation of the surgical object within the seal passage.

7. The surgical apparatus according to claim **6** wherein the outer wall segment of the seal is substantially tubular.

8. The surgical apparatus according to claim **7** wherein the inner seal segment is substantially planar and is arranged in general orthogonal relation with respect to the longitudinal axis.

9. The surgical apparatus according to claim **8** wherein the outer segment and the inner seal segment are monolithically formed.

10. The surgical apparatus according to any of claims **2** to **9** including a fluid disposed within the seal chamber external of the seal, the fluid dimensioned to restrict radial movement of the outer wall segment of the seal
